# EUROPÄISCHE PATENTSCHRIFT

(11) **EP 1 068 009 B1**
(45) Veröffentlichungstag und Bekanntmachung des Hinweises auf die Patenterteilung: **12.12.2001**
(21) Anmeldenummer: 99917827.0
(22) Anmeldetag: 19.03.1999
(51) Int. Cl.: B01J 23/78, B01J 23/83, C07C 17/156, B01J 27/122, B01J 37/00

(54) **PSEUDOBÖHMIT UND GAMMA AL2O3 ENTHALTENDER TRÄGERKATALYSATOR, DESSEN HERSTELLUNG UND DESSEN VERWENDUNG ZUR HERSTELLUNG VON 1,2-DICHLORETHAN**
SUPPORTED CATALYST CONTAINING PSEUDO-BOEHMITE AND GAMMA-AL2O3, PRODUCTION OF SAME AND ITS USE FOR PRODUCING 1,2-DICHLOROETHANE
CATALYSEUR SUPPORTE CONTENANT DE LA PSEUDOBOEHMITE ET GAMMA-AL2O3, SON PROCEDE DE PRODUCTION ET SON UTILISATION DANS LA PRODUCTION DE 1,2-DICHLORETHANE

(30) Priorität: 23.03.1998 DE 19812468
(43) Veröffentlichungstag der Anmeldung: 17.01.2001
(73) Patentinhaber: BASF AKTIENGESELLSCHAFT, 67056 Ludwigshafen (DE)
(72) Erfinder: HEBGEN, Werner, D-69124 Heidelberg (DE); RIEKER, Christopher, William, D-67065 Ludwigshafen (DE); MEISSNER, Ruprecht, D-67273 Weisenheim (DE)
(86) Internationale Anmeldenummer: EP9901826
(87) Internationale Veröffentlichungsnummer: WO9948606

(56) Entgegenhaltungen:
- EP-A- 0 240 714
- DE-A- 3 334 223
- DE-A- 4 311 650

## Beschreibung

Gegenstand der vorliegenden Erfindung ist ein Trägerkatalysator für die Herstellung von 1,2-Dichlorethan durch Umsetzung von etwa 2 Molen Ethylen, etwa 4 Molen Chlorwasserstoff und etwa einem Mol Sauerstoff in Gegenwart eines als Festbett vorliegenden Trägerkatalysators auf der Grundlage von Kupfer (II)-chlorid und Kaliumchlorid auf einem Trägermaterial auf Basis von Aluminiumoxid.

Weiterhin betrifft die Erfindung die Verwendung dieses Trägerkatalysators zur Herstellung von 1,2-Dichlorethan

Die Herstellung von 1,2-Dichlorethan (EDC) ist ein Zwischenschritt bei der Herstellung von monomerem Vinylchlorid, Dazu werden Ethylen, Chlorwasserstoff und Sauerstoff oder ein Sauerstoff enthaltendes Gas meist im stöchiometrischen Verhältnis umgesetzt, wobei Trägerkatalysatoren verwendet werden, die Kupfer (II) chlorid als aktive Komponente enthalten. Dieses Verfahren wird auch als Oxychlorierung bezeichnet. Es haben sich insbesondere zwei Verfahren in der Technik eingeführt, bei denen man den Katalysator entweder als Festbett anordnet oder die Reaktion im Fließbett betreibt.

Das Fließbettverfahren zur Herstellung von EDC erlaubt eine gute Wärmeabfuhr, wodurch hohe Umsatzmengen pro Volumeneinheit Katalysator bei relativ niedrigen Prozeßtemperaturen möglich sind. Nachteilig ist bei diesem Verfahren, daß es zum Agglomerieren der Katalysatorteilchen und Zusammenbruch des Wirbelbetts kommen kann. Weiterhin muß bei diesem Verfahren das bei der Reaktion gebildete Wasser durch eine Fällungsreaktion von Kupferionen befreit werden, welche aus Katalysatorstaub stammen, der im Austrag mitgerissen wird.

Bei dem Festbettverfahren vermeidet man alle Nachteile des Fließbettverfahrens, doch entstehen Probleme beim Wärmetransport und der Beherrschung der Reaktionstemperaturen. Man kennt im wesentlichen einstufige Verfahren, bei denen die Reaktionspartner zur Herstellung von EDC in einer Reaktionszone umgesetzt werden oder auch dreistufige Verfahren mit drei Reaktionszonen, bei denen man Sauerstoff und ggf. andere Reaktionsteilnehmer nicht nur am Einlaß der ersten Reaktionszone, sondern auch am Einlaß der weiteren Reaktionszonen zufährt. Man vermeidet dadurch die Verwendung von Gemischen aus Ethylen, Chlorwasserstoff und Sauerstoff, deren Sauerstoffgehalt im zündfähigen Bereich liegt. Solche dreistufigen Verfahren haben jedoch den Nachteil des relativ komplizierten apparativen Aufwands.

Bei den einstufigen Verfahren treten die Nachteile des Festbettverfahrens in stärkerem Maße auf, so daß man durch kompliziert angeordnete Aktivitätsprofile des Katalysatorbettes versucht hat, ausgeglichene Temperaturen in der Reaktionszone einzuhalten.

In DE-A-33 34 223 wird ein Verfahren zur Herstellung von EDC mit reinem Sauerstoff und Abgasrückführung beschrieben, bei dem der Ethylengehalt des in die Reaktionszone eintretenden Gasgemisches derart geregelt wird, daß das Abgas unter 20 Vol.-% Ethylen enthält. Auf diese Weise wurde eine gute Ausbeute an 1,2-Dichlorethan in Bezug auf das eingesetzte Ethylen und den Chlorwasserstoff erreicht, wobei aufgrund der Verwendung von nur einer Reaktionszone, also eines Reaktors, ein komplizierter apparativer Aufwand nicht erforderlich ist. Ein ähnliches Verfahren beschreibt die EP-A 240 714.

Die in DE-A-33 34 223 und EP-A 240 714 eingesetzten Trägerkatalysatoren enthalten als Trägermaterial Al₂O₃. Von Al₂O₃ sind jedoch verschiedene Modifikationen bekannt, die sich hinsichtlich ihrer Struktur und ihrer mechanischen Eigenschaften stark unterscheiden und hinsichtlich ihrer Eignung als Trägermaterial für Oxychlorierungskatalysatoren starke Unterschiede aufweisen. Im allgemeinen lassen Katalysatoren auf der Basis von Al₂O₃ entweder hinsichtlich ihrer mechanischen Belastbarkeit und Abriebfestigkeit oder hinsichtlich ihrer Produktivität und Selektivität noch zu wünschen übrig.

Der vorliegenden Erfindung lag daher die Aufgabe zugrunde, einen Trägerkatalysator bereitzustellen, der bei der Herstellung von 1,2-Dichlorethan eine gute Produktivität und Selektivität aufweist und dabei eine gute Abriebfestigkeit zeigt.

Diese Aufgabe wird durch den Trägerkatalysator nach Anspruch 1 gelöst.

Bei der Herstellung von 1,2-Dichlorethan werden die Reaktionsteilnehmer, also Ethylen, Chlorwasserstoff und Sauerstoff, zweckmäßig in stöchiometrischen Mengen dem Mischsystem zugeführt, d.h. man mischt Ethylen, Chlorwasserstoff und Sauerstoff in einem solchen Verhältnis, daß auf etwa 1 Mol Sauerstoff etwa 2 Mole Ethylen und etwa 4 Mole Chlorwasserstoff entfallen. Dies soll heißen, daß die Mengen innerhalb eines Bereiches von ± 10 %, bezogen auf die angegebene Molmenge, schwanken können. Nach dem erfindungsgemäßen Verfahren mischt man also das Abgas, also die nichtkondensierbaren Prozeßgase, die unter 20 Vol.-% Ethylen, insbesondere 0,1 bis 5 Vol.-% Ethylen, und noch etwas Sauerstoff, meistens zwischen 0,5 und 1,5 Vol.-%, enthalten, dem Frischgas, das die obige Zusammensetzung hat, zu. Zunächst wird Ethylen und Chlorwasserstoff in einem Mischaggregat mit dem Abgas gemischt. Dann wird dem Gemisch Sauerstoff zugegeben und zwar derart, daß das sauerstoffhaltige Gemisch, das zündfähig ist, nur eine geringe räumliche Ausdehnung innerhalb der Misch-Apparatur hat und der Sauerstoffgehalt der homogenen Mischung nicht höher als 7 Vol.-% ist. Dieser Wert wird mit einem Sauerstoffanalysator laufend überwacht. Das resultierende Gemisch ist aufgrund seines niedrigen Sauerstoffgehaltes unter den Reaktionsbedingungen nicht mehr zündfähig. Diese gesamte Mischung wird dann dem Reaktor zugeführt.

Der Ethylengehalt des in die Reaktionszone eintretenden Gasgemisches wird derart geregelt, daß das Abgas unter 20 Vol.-% Ethylen enthält. Unter Abgas versteht man die nicht kondensierbaren Anteile des Reaktionsgemisches nach dem Abtrennen des 1,2-Dichlorethans und des Wassers. Die Regelung erfolgt derart, daß beim Ansteigen des Ethylengehaltes im Abgas der Ethylenstrom an frischem Ethylen reduziert und bei einem Abfallen unter 0,1 Vol.-% angehoben wird. Die Menge des zurückgeführten Abgases wird in Abhängigkeit von dessen Sauerstoffgehalt, meistens zwis-chen 0,5 bis 1,5 Vol.-%, so eingestellt, daß der nach der Mischung mit den Einsatzstoffen Chlorwasserstoff, Ethylen und Sauerstoff kontinuierlich gemessene Sauerstoffgehalt bei 7 Vol.-% gehalten wird.

Nach einer energetisch besonders vorteilhaften Arbeitsweise entnimmt man das zurückgeführte Kreisgas dem gasförmigen Strom nach dem wassergekühlten Kondensator und führt nur noch den wesentlich kleineren Teil des Abgases, der aus dem System ausgeschleust wird, über einen Solekühler, um weitere Anteile von 1,2-Dichlorethan und Wasser zu kondensieren. Das somit bei höherer Temperatur anfallende und deshalb partialdruckmäßig mit 1,2-Dichlorethan und Wasser höher angereicherte Kreisgas hat unerwartet keinen negativen Einfluß auf die Reaktion.

Nach einer weiteren besonders vorteilhaften Arbeitsweise zweigt man aus den Abgasen 0,5 bis 20 Vol.-% ab, führt diesen abgezweigten Anteil über eine Kohlendioxidabsorptionskolonne und leitet sie dann wiederum dem Abgasstrom zu. Nach dieser Arbeitsweise erreicht man, daß noch geringere Ethylenverluste bei dem Verfahren auftreten.

Weitere Einzelheiten über eine geeignete Vorrichtung zur Durchführung des Oxychlorierungsverfahrens sowie über vorteilhafte Verfahrensvarianten werden in DE-A-3334223 beschrieben.

Der erfindungsgemäße Katalysator basiert auf einen Träger, welcher aus einer Mischung von Pseudoböhmit und γ-Al₂O₃ hergestellt wird. Diese beiden Komponenten werden in feinpulveriger Form miteinander vermischt, wobei das Gewichtsverhältnis zwischen 4:1 und 1:4 liegt, vorzugsweise zwischen 1:1 und 1:3. Dem Gemisch werden vorzugsweise noch Tablettierungsmittel, die vor allem als Gleitmittel dienen, zugegeben. Dem Fachmann sind viele derartige Tablettierungshilfsmittel bekannt. Genannt seien hier nur beispielhaft Magnesiumstearat und Graphit. Magnesiumstearat wird vorzugsweise in Mengen von 0,5 bis 7 Gew.-%, besonders bevorzugt von 2 bis 5 Gew.-%, bezogen auf die Gesamtmasse der Mischung, zugeben. Graphit wird im allgemeinen in einer Menge von 0,5 bis 3 Gew.-%, vorzugsweise von 1 bis 1,5 Gew.-% zugegeben.

Die so erhaltene Mischung wird anschließend tablettiert. Vorzugsweise wird das Material in Ring- oder Zylinderform gepresst. Die Ringe haben vorzugsweise etwa 5 bis 7 mm Außendurchmesser, 2 bis 3 mm Innendurchmesser und 3 bis 8 mm Höhe, die Zylinder vorzugsweise einen Durchmesser von 3 bis 7 mm und eine Höhe von ebenfalls 3 bis 7 mm.

Zur Tablettierung werden die üblichen Pressen verwendet, die Preßkraft beträgt vorzugsweise mehr als 9 kN, besonders bevorzugt zwischen 9 und 11 kN.

Nach der Tablettierung werden die Katalysatorträger calciniert. Die Calcinierung wird im allgemeinen für 0,5 bis 10 h bei 500 bis 800°C durchgeführt, vorzugsweise für ca. 2 h bei 700 bis 750°C. Die Calcinierung wird in oxidierender Atmosphäre, im allgemeinen an Luft vorgenommen.

Durch das beschriebene Tablettierungsverfahren und die Auswahl der Einsatzstoffe wird ein Katalysatorträger erhalten, der einerseits eine gute mechanische Stabilität und andererseits ein für die Oxychlorierung optimales Porenvolumen aufweist. Das Porenvolumen ist so groß, daß eine große innere Oberfläche für die Aktivkomponente bereitgestellt wird. Auf der anderen Seite ist das Porenvolumen jedoch nicht zu groß, da sonst die mechanische Stabilität leiden würde und außerdem keine optimale Schüttdichte im Reaktor erzielt würde. Die erfindungsgemäß erhaltenen Katalysatorträger haben vorzugsweise ein Porenvolumen von 0,3 bis 0,7 cm³/g, besonders bevorzugt von 0,4 bis 0,6 cm³/g.

Der so erhaltene Träger wird anschließend mit einer CuCl₂/KCl-Lösung getränkt. Die Tränklösung kann auch noch HCl sowie Salze der Elemente Li, Na, Cs, Mg, Ca sowie der seltenen Erdmetalle, vorzugsweise der Chloride dieser Elemente enthalten. Der pH-Wert der Tränklösung beträgt vorzugsweise 6 bis 8.

Das Volumen der Tränklösung wird vorteilhaft so gewählt, daß es 10 bis 200 % des Porenvolumens des Trägers entspricht, besonders bevorzugt 90 bis 110 %.

Nach der Tränkung werden die Katalysatorformlinge getrocknet, im allgemeinen für 0,2 bis 10 h bei 80 bis 300°C, vorzugsweise für 0,5 bis 2 h bei 100 bis 200°C.

Die Konzentration und das Volumen der Tränklösung werden so gewählt, daß der Trägerkatalysator einen Cu-Gehalt von 1 bis 15 Gew.-%, vorzugsweise von 2 bis 10 Gew.-% und ein K-Gehalt von 0,1 bis 8 Gew.-%, vorzugsweise von 0,3 bis 3 Gew.-% aufweist. Werden auch noch andere Elemente der oben genannten Gruppe auf den Träger aufgetragen, so sollte das Verhältnis Cu: Summe anderer Elemente 1,0 bis 10 mol/mol, vorzugsweise 1,1 bis 6 mol/mol betragen. Für das Cu:K-Verhältnis sind die gleichen Verhältniszahlen bevorzugt. Durch die Wahl der Metallkonzentrationen im Katalysator sowie gewünschtenfalls durch Verdünnung mit Inertmaterial wie Al₂O₃ kann das Aktivitätsprofil der Katalysatorpräparation wunschgemäß eingestellt werden. Der auf diese Weise erhältliche Trägerkatalysator eignet sich in hervorragender Weise zur Verwendung im Verfahren zur Herstellung von 1,2-Dichlorethan durch Oxychlorierung. Er verbindet ausgezeichnete Produktivität und Selektivität mit guter mechanischer Belastbarkeit und erlaubt so lange Standzeiten.

Die folgenden Beispiele erläutern die Erfindung.

### Beispiele

### Herstellung der Trägertabletten

Eine Trockenmischung von 6 kg gamma-Aluminiumoxid (Puralox SCF a230, Fa. Condea), 4 kg Pseudoböhmit (Pural SCF; Fa. Condea), 324 g Magnesiumstearat und 100 g Graphit wurden zu Ringtabletten der Dimension 5 mm x 5mm x 2 mm (Höhe x Außendurchmesser x Innendurchmesser) mittels einer Tablettenpresse der Fa. Kilian (Model LX 18) auf eine Seitendruckfestigkeit der Tabletten von 25 N verpreßt. Anschließend wurden die Tabletten unter normaler Luftacmosphäre für 2 Stunden bei einer Temperatur von 700°C calciniert. Die so hergestellten Tabletten wiesen folgende physikalische Eigenschaften auf:

| | |
|---|---|
| Litergewicht | 705 g/l |
| Wasseraufnahme | 0,51 ml/g |
| Seitendruckfestigkeit | 33 N |
| BET-Oberfläche | 200 m²/g |
| Glühverlust bei 900°C | 4,0 Gew.-% |

### Herstellung des geträgerten Katalysators

Für die Befüllung des Reaktors wurden Katalysatortypen unterschiedlicher Aktivität durch Tränken der Trägertabletten mit wässriger CuCl₂/KCl-Lösung unterschiedlicher Zusammensetzung hergestellt. Die CuCl₂-Konzentration der Tränklösung wurde so eingestellt, daß Kupfergehalte der Katalysatoren zwischen 1,7 Gew.-% und 5,5 Gew.-% erhalten wurden. Als Vergleichskatalysator wurde ein Trägermaterial nach DE-A-3334223 mit den entsprechenden Tränklösungen behandelt.

### Befüllung des Reaktors

Der in verschiedenen Aktivitätsstufen hergestellte Katalysator wurde so in den Reaktor gefüllt, daß der Kupfergehalt am Reaktoreingang 1,7 Gew.-% betrug und bis zum Reaktorausgang auf 5,5 Gew.-% anstieg.

### Herstellung von EDC

Alle Versuche wurden in einer halbtechnischen Oxichlorierungsanlage durchgeführt. Bei allen Versuchen wurde das Kreisgas = rückgeführtes Abgas zuerst mit Ethylen und HCl vermischt und anschließend Sauerstoff zugegeben. Der Sauerstoffgehalt am Reaktoreingang lag bei etwa 7 % Sauerstoff oder darunter. Das den Reaktor verlassende Gasgemisch wurde zunächst in einem mit Wasser betriebenen Kondensator gekühlt und teilweise kondensiert. Nach Abtrennung der kondensierten Bestandteile (Roh-EDC und Wasser) wurde die überwiegende Menge des nicht kondensierten Anteils um etwa 10°C erwärmt und mittels eines Kompressors zum Reaktoreingang gefördert. Eine kleinere Menge wurde druckgeregelt (4,5 bar) über einen mit Sole betriebenen Kühler geleitet. Die dort kondensierten Anteile wurden mit dem Kondensat nach dem Wasserkühler vereirigt, das Abgas wurde abgeleitet. Die Temperaturen im Reaktor lagen zwischen 180 und 270°C.

Der Vergleichsversuch V1 gibt den Stand der Technik nach der EP-A 240 714 und DE-A 33 34 223 wieder. Der verwendete Katalysator hatte nur gamma-Aluminiumoxid als Trägermaterial. Die Versuch A, B und C stellen drei unabhängige Versuchsreihen dar und sind erfindungsgemäß, d.h. der oben beschriebene verbesserte Katalysator wurde eingesetzt.

In den beiliegenden Tabellen sind die EDC-Produktion sowie die Umsätze von Ethen und HCl als durchschnittliche Werte über mehrere Einzelbestimmungen zusammengestellt. Die Versuchsdauer lag jeweils bei 4 bis 10 Wochen. Nach 6 Wochen wurde der Druckanstieg, der auf Katalysatorabrieb zurückgeführt werden kann, gemessen.

Nach der beiliegenden Tabelle 1 liegen die Vorteile des erfindungsgemäßen Verfahrens gegenüber den Vergleichsversuchen vor allem in der höheren Produktivität, im verringerten spezifischen Abgas und damit besserem Ethenumsatz. Der verbesserte Katalysator zeichnet sich durch eine niedrigere Verbrennungsrate und eine verbesserte Abriebfestigkeit aus, welche zu einem geringeren Anstieg des Druckverlustes DP über den Reaktor führt.

**Tabelle 1:**

| Ergebnisse der Versuche zur EDC-Herstellung mit den erfindungsgemäßen Katalysatoren | | | | |
|---|---|---|---|---|
| Beispiel | EDC-Produktivität kg/h | Umsatz % Ethen | Umsatz % HCl | Druckanstieg nach 6 Wochen in % |
| A | 88,8 | 99,9 | 98,6 | 10,0 |
| B | 84,9 | 99,9 | 98,8 | 7,4 |
| C | 93,0 | 99,9 | 99,1 | 12,9 |
| V1 | 72,9 | 99,8 | 97,9 | 14,3 |

## Patentansprüche

1. Trägerkatalysator für die 1,2-Dichlorethan-Herstellung, erhältlich durch
a) Bereitstellen eines pulverförmigen Trägermaterials auf Basis von Aluminiumoxid,
b) Tablettieren des Trägermaterials durch Verpressen,
c) Calcinieren des Katalysatorträgers 0,5 bis 10 h bei 500 bis 800°C,
d) Tränken des Katalysatorträgers mit einer CuCl₂/KCl-Lösung, so daß der Kupfergehalt 1 bis 15 Gew.-% und der Kaliumgehalt 0,1 bis 8 Gew.-%, jeweils bezogen auf die Gesamtmasse des Katalysators, beträgt,
e) Trocknen des Katalysators bei 60 bis 400°C,
**dadurch gekennzeichnet, daß** als Trägermaterial eine Mischung von γ-Al₂O₃ mit Pseudoböhmit im Gewichtsverhältnis 4:1 bis 1:4, gewünschtenfalls unter Zusatz von Tablettierungshilfsmitteln, eingesetzt wird.

2. Trägerkatalysator nach Anspruch 1, **dadurch gekennzeichnet, daß** das Gewichtsverhältnis von Pseudoböhmit und γ-Al₂O₃ in der Tablettierungsmischung zwischen 1:1 und 1:3 liegt.

3. Trägerkatalysator nach Anspruch 1, **dadurch gekennzeichnet, daß** der Katalysatorträger ein Porenvolumen von 0,3 bis 0,7 cm³/g aufweist.

4. Trägerkatalysator nach Anspruch 1, **dadurch gekennzeichnet, daß** der Cu-Gehalt des Katalysators 2 bis 10 Gew.-% beträgt.

5. Trägerkatalysator nach Anspruch 1, **dadurch gekennzeichnet, daß** der K-Gehalt des Katalysators 0,3 bis 3 Gew.-% beträgt.

6. Trägerkatalysator nach Anspruch 1, **dadurch gekennzeichnet, daß** der Katalysator zusätzlich zu den Elementen Kupfer und Kalium mit Elementen aus der Gruppe Li, Na, Cs, Mg, Ca und den seltenen Erdmetallen beladen wird.

7. Verwendung des Katalysators gemäß Anspruch 1 zur Herstellung von 1,2-Dichlorethan.

## Claims

1. A supported catalyst for preparing 1,2-dichloroethane and obtainable by
a) preparing a pulverulent support material based on aluminum oxide,
b) using compression to tablet the support material,
c) calcining the catalyst support for from 0.5 to 10 h at from 500 to 800°C,
d) saturating the catalyst support with a CuCl₂/KCl solution so that the copper content is from 1 to 15% by weight and the potassium content is from 0.1 to 8% by weight, in each case based on the total weight of the catalyst,
e) drying the catalyst at from 60 to 400°C,
wherein the support material used comprises a mixture of γ-Al₂O₃ with pseudoboehmite in a weight ratio of from 4:1 to 1:4, if desired with addition of tableting auxiliaries.

2. A supported catalyst as claimed in claim 1, wherein the ratio by weight of pseudoboehmite and γ-Al₂O₃ in the tableting mixture is from 1:1 to 1:3.

3. A supported catalyst as claimed in claim 1, wherein the catalyst support has a pore volume of from 0.3 to 0.7 cm³/g.

4. A supported catalyst as claimed in claim 1, wherein the Cu content of the catalyst is from 2 to 10% by weight.

5. A supported catalyst as claimed in claim 1, wherein the K content of the catalyst is from 0.3 to 3% by weight.

6. A supported catalyst as claimed in claim 1, wherein the catalyst is loaded with elements selected from the group consisting of Li, Na, Cs, Mg, Ca and the rare earth metals, in addition to the elements copper and potassium.

7. The use of the catalyst as claimed in claim 1 for preparing 1,2-dichloroethane.

## Revendications

1. Catalyseur supporté pour la préparation de 1,2-dichloréthane, que l'on peut obtenir par:
a) préparation d'un matériau de support pulvérulent à base d'oxyde d'aluminium,
b) pastillage du matériau de support par pressage,
c) calcination du support de catalyseur pendant 0,5 à 10 heures à une température de 500 à 800°C,
d) imprégnation du support de catalyseur par une solution de CuCl₂/KCl, de manière que la teneur en cuivre soit de 1 à 15% en poids et la teneur en en potassium de 0,1 à 8% en poids, à chaque fois par rapport à la masse totale du catalyseur,
e) séchage du catalyseur à une température de 60 à 400°C,
**caractérisé en ce que** l'on met en oeuvre comme matériau de support un mélange de γ-Al₂O₃ et de pseudoboehmite en rapport pondéral de 4:1 à 1:4, éventuellement avec addition d'adjuvants de pastillage.

2. Catalyseur supporté selon la revendication 1,
**caractérisé en ce que** le rapport pondéral de la pseudoboehmite et du γ -Al₂O₃ dans le mélange de pastillage se situe entre 1:1 et 1:3.

3. Catalyseur supporté selon la revendication 1,
**caractérisé en ce que** le support de catalyseur présente un volume porique de 0,3 à 0,7 cm³.

4. Catalyseur supporté selon la revendication 1,
**caractérisé en ce que** la teneur en Cu du catalyseur est de 2 à 10 en poids.

5. Catalyseur supporté selon la revendication 1,
**caractérisé en ce que** la teneur en K du catalyseur est de 0,3 à 3% en poids.

6. Catalyseur supporté selon la revendication 1,
**caractérisé en ce que** le catalyseur est chargé, outre Les éléments cuivre et potassium, d'éléments du groupe formé par Li, Na, Cs, Mg, Ca et les terres rares.

7. Utilisation du catalyseur selon la revendication 1 pour la préparation de 1,2-dichloréthane.
